# EUROPEAN PATENT APPLICATION

(11) **EP 4 768 036 A1**
(43) Date of publication of application: **01.07.2026**
(21) Application number: 24856446.0
(22) Date of filing: 20.08.2024
(51) Int. Cl.: A61K 35/748, A61K 8/9706, A61K 8/9728, A61K 35/74, A61P 17/16, A61P 17/18, A61P 39/06, A61Q 17/04, A61Q 19/00, C12N 1/13, C12N 1/20, C12N 1/21, C12N 15/31, C12N 15/52, C12N 15/63, C12P 1/04, C12Q 1/02

(54) **ANTIOXIDANT, METHOD FOR REMOVING OXYGEN RADICALS, AND METHOD FOR PRODUCING ANTIOXIDANT**

(30) Priority: 23.08.2023 JP 2023135655; 23.08.2023 JP 2023135656; 23.08.2023 JP 2023135657; 26.07.2024 JP 2024121454
(71) Applicant: Panasonic Intellectual Property Management Co., Ltd., Kadoma-shi, Osaka 571-0057 (JP)
(72) Inventor: OHYAMA Tatsushi, Kadoma-shi, Osaka 571-0057 (JP); KUSAMA Shoko, Kadoma-shi, Osaka 571-0057 (JP); KOJIMA Seiji, Kadoma-shi, Osaka 571-0057 (JP); WAKABAYASHI Masaya, Kadoma-shi, Osaka 571-0057 (JP); KIRIMURA Makoto, Kadoma-shi, Osaka 571-0057 (JP)
(74) Representative: Grünecker Patent- und Rechtsanwälte PartG mbB
(86) International application number: PCT/JP2024/029450
(87) International publication number: WO 2025/041758

(57) **Abstract**

The present disclosure provides a novel antioxidant using cyanobacteria. An antioxidant of the present disclosure includes a cyanobacterial secretion.

## Description

### BACKGROUND

### 1. Technical Field

The present disclosure relates to an antioxidant, a method for removing active oxygen, and a method for producing an antioxidant.

### 2. Description of the Related Art

Attempts have been made to obtain useful components using cyanobacteria.

For example, Patent Literature 1 describes a method for obtaining a water-soluble compound such as phycocyanin from cyanobacteria. In this method, cyanobacteria mixed with an aqueous solution are subjected to ultrasonic treatment under specific conditions to extract the water-soluble compound.

Patent Literature 2 describes polysaccharides released from Cyanothece sp. CCY 0110 for pharmaceutical, veterinary or cosmetic use. The Cyanothece sp. CCY 0110 is one kind of cyanobacteria and is a very efficient producer of extracellular polymeric substances, releasing up to 75% of the polymer into a culture medium. The release kinetics of polysaccharide from Cyanothece sp. CCY 0110 is modulated by the addition of divalent cations.

Patent Literature 3 describes a method for producing a mycosporine-like amino acid. This method comprises a step of culturing a microorganism that extracellularly produces a mycosporine-like amino acid, a step of separating cells from an extracellular culture solution, and a step of recovering the mycosporine-like amino acid from the extracellular culture solution. The microorganism is Escherichia coli, yeasts, actinomycetes, microalgae, or a microorganism belonging to Labyrinthulomycetes.

Non-Patent Literature 1 describes a promoter that enables efficient production of heterologous proteins in cyanobacteria.

### Citation List

### Patent Literature

Patent Literature 1: WO 2018/229364
Patent Literature 2: WO 2018/042378
Patent Literature 3: WO 2015/174427

### Non-Patent Literature

Non-Patent Literature 1: Jie Zhou et al., "Discovery of a super-strong Scientific Reports, Research, 2014, Vol.4, Article No. 4500 (2014)

### SUMMARY

### (Technical Problem)

The techniques described in the above literatures have room for review from the viewpoint of obtaining antioxidants using cyanobacteria. Thus, the present disclosure provides a novel antioxidant using cyanobacteria.

### (Solution to Problem)

The present disclosure provides an antioxidant comprising a secretion of a cyanobacterium.

### (Advantageous Effects of Invention)

The antioxidant of the present disclosure is a novel antioxidant that can be obtained using cyanobacteria.

### BRIEF DESCRIPTION OF DRAWINGS

FIG. 1 is a flowchart showing an example of a method for producing an antioxidant according to the present embodiment.
FIG. 2 is a diagram schematically showing an example of a gene sequence of a vector DNA used for genetic modification of cyanobacteria.
FIG. 3 is a diagram showing results of electrophoresis of DNA fragments amplified from modified cyanobacteria according to the inventive example.
FIG. 4 is a graph showing measurement results of activity values of lipopolysaccharide (LPS) in culture supernatants according to the inventive example and the comparative example.
FIG. 5 is a graph showing change over time of difference in absorbance in samples for evaluation of antioxidant capacity according to the inventive example and the comparative example.
FIG. 6A is a graph showing a temporal change in skin moisture content when sample for moisturizing properties evaluation according to the inventive example and pure water are used.
FIG. 6B is a graph showing a temporal change in skin moisture content when sample for moisturizing properties evaluation according to the comparative example and pure water are used.
FIG. 7A is a graph showing a temporal change in skin moisture content when sample for moisturizing properties evaluation according to the inventive example and pure water are used.
FIG. 7B is a graph showing a temporal change in skin moisture content when sample for moisturizing properties evaluation according to the comparative example and pure water are used.
FIG. 8 is a graph showing ultraviolet absorption spectra of a culture supernatant liquid according to the inventive example and the comparative example.
FIG. 9A is a graph showing a relationship between a cotyledon development ratio of pansy when irrigated with the culture supernatant liquid according to the inventive example or pure water and the number of days elapsed from sowing of pansy seeds.
FIG. 9B is a graph showing a relationship between a cotyledon development ratio of spinach when irrigated with the culture supernatant liquid according to the inventive example or pure water and the number of days elapsed from seeding of spinach seeds.

### DESCRIPTION OF EMBODIMENT

### (Findings Underlying the Present Disclosure)

For example, antioxidants are expected to prevent skin aging and aging of living bodies in cosmetics, quasi drugs, pharmaceuticals, foods, and beverages, which appeal to reduce skin or living body deterioration, and improve skin or living body quality. For example, antioxidants are expected to prevent skin troubles such as spots, wrinkles, and sagging.

It is becoming clear that oxidation of biological tissues by reactive oxygen species is closely involved in lifestyle-related diseases such as cancer, arteriosclerosis, and heart disease or aging. The active oxygen species is a generic term for highly reactive compounds containing oxygen. Examples of the active oxygen species are hydroxy radical (•OH), alkoxy radical (LO•), peroxy radical (LOO•), hydroperoxy radical (HOO•), nitric oxide (NO), nitrogen dioxide (NO₂), superoxide anion (O₂⁻), ozone (O₃), hydrogen peroxide (H₂O₂), and lipid hydroperoxide (LOOH). These reactive oxygen species have high reactivity, oxidize biological substances such as proteins, lipids, and DNA in living organisms, cause protein denaturation, lipid peroxidation, and gene damage, and are considered to be involved in the onset of various diseases.

The reactive oxygen species can be removed by ascorbic acid, tocopherol, carotenoid, and various polyphenols. Therefore, it is expected that the use of antioxidants may be effective for the prevention of various diseases associated with active oxygen.

A composition in which a chemical synthetic substance is formulated so as to obtain a desired effect and efficacy is intended to improve the appearance or eliminate troubles in a short-term approach, and is made on the basis of Western medical principles. On the other hand, it is conceivable to produce an organic antioxidant on the basis of the Oriental medical principles that the antioxidant has a composite effect and efficacy derived from a plant, does not impair the original function of a human, and enhances self-healing ability or a barrier function for a long period of time. A composition containing a chemically synthesized substance may cause a problem of irritation to the skin or living body, and organic products tend to be preferred. In the markets of cosmetics, quasi-drugs, pharmaceuticals, foods, and beverages, products derived from nature continue to grow in recent years, and interest in products appealing to nature or organic is increasing. However, it is not easy to make organic antioxidants. Microorganisms often do not produce large amounts of the compound of interest, making it difficult to obtain commercially meaningful quantities of the compound using microorganisms.

Cyanobacteria have recently attracted the attention of many researchers and operators. Cyanobacteria contain a number of value-added compounds and many researchers and operators are interested in extracting a compound such as phycocyanin from cyanobacteria. Meanwhile, in order to use a compound produced in a wild type cyanobacterium, it is necessary to crush the cyanobacterium and extract the compound. According to the technique described in Patent Literature 1, it is necessary to subject cyanobacteria mixed with an aqueous solution to ultrasonic treatment under predetermined conditions, and it is considered that the process for obtaining a target compound is complicated and the production cost of a composition containing the compound is likely to increase. According to the technique described in Patent Literature 2, a polysaccharide released from Cyanothece sp. CCY 0110 is obtained, but it is not certain whether a component having an antioxidant effect is obtained. It will also be understood that there is a limit to the amount of polysaccharide released from Cyanothece sp. CCY 0110. According to the technique described in Patent Literature 3, although a mycosporine-like amino acid can be obtained, it is unclear whether a component having an antioxidant effect can be obtained. According to the technique described in Non-Patent Literature 1, it is expected that a desired protein is produced inside a cell of cyanobacteria. However, the protein produced inside the cell of the cyanobacterium is hardly excreted to the outside of the cell, and in order to obtain the protein, it is necessary to crush the cell of the cyanobacterium and extract the protein produced inside the cell.

As a result of intensive studies, the present inventors have newly found that a secretion of cyanobacteria can be obtained by a specific method so as to have an antioxidant effect. Based on this new finding, the present inventors have completed the antioxidant of the present disclosure.

Cosmetics, quasi drugs, and pharmaceuticals, which appeal to reduce skin deterioration and improve skin quality, may be required to have an effect of preventing inflammation, darkening, and dryness caused on the skin by ultraviolet rays such as UV-A and UV-B. UV-A is ultraviolet radiation that quickly darkens the skin after sunburn. It can pass through clouds and window glass, reach the dermis deep in the skin, and cause photoaging of the skin such as wrinkles and sagging. UV-B is an ultraviolet radiation which mainly acts strongly on the epidermis, causing red irritation of the skin several hours after exposure to the sun. UV-B increases melanin and can cause sunburn spots or freckles. Such repeated sunburn also leads to photoaging of the skin. In addition, when a person is exposed to ultraviolet rays included in sunlight, active oxygen is generated inside human cells, and skin oxidation and skin aging may occur. Therefore, in order to protect the skin from ultraviolet rays, it is conceivable to use a topical dermatological composition having at least one effect selected from the group consisting of an antioxidant effect and an ultraviolet absorbing effect.

As the topical dermatological composition, a composition in which a chemically synthesized substance is blended so as to obtain desired effects and efficacies is considered. Compositions containing such chemically synthesized substances are intended to improve the appearance or eliminate troubles in a short-term approach, and are made based on Western medical principles. On the other hand, it is also conceivable to prepare an organic topical dermatological composition based on the Oriental medical concept, which has composite effects and efficacies derived from plants and is intended for a long period of time so as to enhance self-healing ability or barrier function without impairing the original functions of humans. A composition containing a chemically synthesized substance may cause a problem of skin irritation, and an organic skin care agent tends to be preferred. In the market of cosmetics, quasi drugs, and pharmaceuticals, which are used for external application to the skin, products derived from nature continue to grow in recent years, and interest in topical dermatological composition, which appeal to nature or organics, is increasing. However, it is not easy to produce organic products for external application to the skin. Microorganisms often do not produce large amounts of the compound of interest, making it difficult to obtain commercially meaningful quantities of the compound using microorganisms.

According to the technique described in Patent Literature 1, it is necessary to subject cyanobacteria mixed with an aqueous solution to ultrasonic treatment under predetermined conditions, and it is considered that the process for obtaining a target compound is complicated and the production cost of a composition containing the compound is likely to increase. According to the technique described in Patent Literature 2, a polysaccharide released from Cyanothece sp. CCY0110 can be obtained, but it is not certain whether a composition having predetermined effects such as an antioxidant effect and an ultraviolet absorbing effect can be obtained. It will also be appreciated that there is a limit to the amount of polysaccharide released from Cyanothece sp. CCY 0110. According to the technique described in Patent Literature 3, a mycosporine-like amino acid can be obtained, but it is not certain whether a composition having predetermined effects such as an antioxidant effect and an ultraviolet absorbing effect can be obtained. According to the technique described in Non-Patent Literature 1, it is expected that a desired protein is produced inside a cell of cyanobacteria. However, the protein produced inside the cell of the cyanobacterium is hardly excreted to the outside of the cell, and in order to obtain the protein, it is necessary to crush the cell of the cyanobacterium and extract the protein produced inside the cell.

As a result of intensive studies, the present inventors have newly found that a secretion of cyanobacteria can be obtained by a specific method so as to have a predetermined effect. Based on this new finding, the present inventors have completed the topical dermatological composition of the present disclosure.

In cosmetics, quasi drugs, and pharmaceuticals, which appeal to reduce skin deterioration and improve skin quality, for example, moisturizers are important for preventing a decrease in skin barrier function and maintaining a healthy skin condition. When the barrier function of the skin decreases, the moisture of the stratum corneum cannot be sufficiently maintained, and the skin becomes dry and susceptible to external stimuli such as ultraviolet rays. Irritated skin is prone to various troubles and may become sensitive. Moisturizing properties is important for sensitive skin to prevent a decrease in the barrier function of the skin and to maintain a healthy skin condition.

In addition to moisturizing, it is also important to protect the skin from ultraviolet rays by ultraviolet absorption. The moisturizer may be required to have an effect of preventing inflammation, blackening, and dryness caused on the skin by ultraviolet rays such as UV-A and UV-B. UV-A is ultraviolet radiation that quickly darkens the skin after sunburn. It can pass through clouds and window glass, reach the dermis deep in the skin, and cause photoaging of the skin such as wrinkles and sagging. UV-B is an ultraviolet radiation which mainly acts strongly on the epidermis, causing red irritation of the skin several hours after exposure to the sun. UV-B increases melanin and can cause sunburn spots or freckles. Such repeated sunburn also leads to photoaging of the skin.

When a person is exposed to ultraviolet rays included in sunlight, active oxygen is generated inside human cells, and skin oxidation and skin aging may occur. Therefore, in addition to moisturization, antioxidation is also important. Antioxidation is important to prevent accelerated skin aging that can cause skin problems such as spots, wrinkles, and sagging.

The moisturizer may be a composition in which a chemical synthetic substance is blended so as to obtain a desired effect. Compositions containing such chemically synthesized substances are intended to improve the appearance or eliminate troubles in a short-term approach, and are made based on Western medical principles. On the other hand, it is also conceivable to produce an organic moisturizer based on the Oriental medical concept, which has composite effects derived from plants, does not impair the original functions of humans, and enhances self-healing ability or barrier function for a long period of time. Compositions containing chemically synthesized substances may cause skin irritation, and organic products tend to be preferred. In the markets of cosmetics, quasi drugs, and pharmaceuticals, products derived from nature continue to grow in recent years, and interest in moisturizers appealing to nature or organic substances is increasing. However, it is not easy to make organic moisturizers. Microorganisms often do not produce large amounts of the compound of interest, making it difficult to obtain commercially meaningful quantities of the compound using microorganisms.

According to the technique described in Patent Literature 1, it is necessary to subject cyanobacteria mixed with an aqueous solution to ultrasonic treatment under predetermined conditions, and it is considered that the process for obtaining a target compound is complicated and the production cost of a composition containing the compound is likely to increase. According to the technique described in Patent Literature 2, a polysaccharide released from Cyanothece sp. CCY0110 is obtained, but it is not certain whether a component having a moisturizing effect is obtained. It will also be appreciated that there is a limit to the amount of polysaccharide released from Cyanothece sp. CCY 0110. According to the technique described in Patent Literature 3, although a mycosporine-like amino acid can be obtained, it is unclear whether a component having a moisturizing effect can be obtained. According to the technique described in Non-Patent Literature 1, it is expected that a desired protein is produced inside a cell of cyanobacteria. However, the protein produced inside the cell of the cyanobacterium is hardly excreted to the outside of the cell, and in order to obtain the protein, it is necessary to crush the cell of the cyanobacterium and extract the protein produced inside the cell.

As a result of intensive studies, the present inventors have newly found that a secretion of cyanobacteria can be obtained by a specific method so as to have a moisturizing effect. Based on this new finding, the present inventors completed the moisturizer of the present disclosure.

### (Embodiment of the Present Disclosure)

Hereinafter, an embodiment of the present disclosure will be described with reference to the drawings. Each of the embodiments described below shows a general or specific example. The numerical values, shapes, materials, structural elements, the arrangement and connection of the structural elements, process conditions, steps, and the processing order of the steps shown in the following embodiments are mere examples, and therefore do not limit the present disclosure. Therefore, among the structural elements in the following embodiments, structural elements not recited in any one of the independent claims are described as arbitrary structural elements. Note that the drawings are schematic diagrams and are not necessarily precise illustrations. In the drawings, substantially the same components are denoted by the same reference numerals, and overlapping description may be omitted or simplified. In the following description, numerical ranges do not represent only strict meanings but include substantially equivalent ranges. In the present specification, each of the bacterial body and the cell represents an individual of one cyanobacterium.

### (Embodiment)

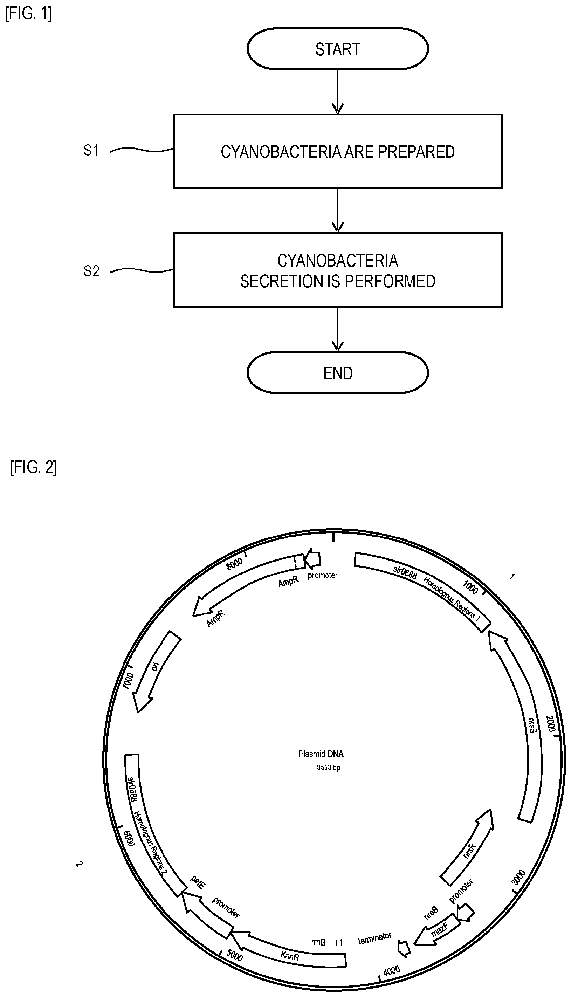

Hereinafter, embodiments will be specifically described with reference to FIGS. 1 and 2. In the present specification, the homology of nucleotide sequences and amino acid sequences is calculated by the Basic Local Alignment Search Tool (BLAST) algorithm. Specifically, it is calculated by performing pairwise analysis with the BLAST program available on the website of the National Center for Biotechnology Information (NCBI). Information on the nucleotide sequence of a gene and the amino acid sequence of a protein encoded by the gene is published on, for example, the NCBI website described above, and can be referred to as needed.

### (Antioxidants)

The antioxidant according to the present embodiment contains a secretion of cyanobacteria. In the present specification, the secretion refers to a substance that is excreted to the outside of the cyanobacteria by the action of the cyanobacteria themselves. Therefore, when a substance that is produced inside cyanobacteria and remains inside cyanobacteria is obtained by an artificial method such as disruption of cyanobacteria, the substance does not correspond to the secretion in the present specification. Having an antioxidant effect means, for example, that the degree of the antioxidant effect is larger than that in a case where an antioxidant is not used.

Cyanobacteria are also called blue-green algae or cyanobacteria, and are a group of prokaryotes that capture light energy with chlorophyll, split water with the obtained energy, and perform photosynthesis while generating oxygen. Cyanobacteria are highly diverse and include, for example, in terms of cell shape, there are unicellular species such as Synechocystis sp. PCC6803 and filamentous species such as Anabaena sp. PCC7120, in which many cells are connected. In terms of growth environments, cyanobacteria also include thermophilic species such as Thermosynechococcus elongatus, marine species such as Synechococcus elongatus, and freshwater species such as Synechocystis. In addition, there are many species that have gas vesicles and produce toxins, such as Microcystis aeruginosa, and species that have unique characteristics, such as Gloeobacterviolaceus, which does not have thylakoids and has a protein called phycobilisome, which is a light-collecting antenna, in the plasma membrane.

The antioxidant includes, for example, secretions excreted to the outside of cyanobacteria, but does not include cyanobacteria bodies or components derived from cyanobacteria bodies. In other words, the antioxidant contains, for example, only secretions excreted to the outside of cyanobacteria as components derived from cyanobacteria.

The antioxidant may further have at least one effect selected from the group consisting of a moisturizing effect and an ultraviolet absorbing effect. In the present specification, having a moisturizing effect means that, for example, the degree of the moisturizing effect is larger than that in a case where an antioxidant is not used. In the present specification, having an ultraviolet absorbing effect means that, for example, the degree of the ultraviolet absorbing effect is larger than that in a case where an antioxidant is not used.

When an antioxidant has an ultraviolet absorbing effect in addition to an antioxidant effect, the antioxidant can contribute to improving the high temperature tolerance and stress tolerance of a plant. For this reason, antioxidants can be used to improve the heat tolerance and stress tolerance of plants.

The secretions contained in the antioxidant are, for example, secretions of modified cyanobacteria. The modified cyanobacteria are, for example, genetically modified so as to allow the secretion to be excreted outside the cyanobacteria. For example, the modified cyanobacteria can excrete, as secretions, compounds that are difficult to excrete by wild-type cyanobacteria that have not undergone genetic modification. The modified cyanobacteria are, for example, genetically modified to allow a component having an antioxidant effect to be excreted.

The modified cyanobacterium is a wild-type cyanobacterium in which the genomic DNA inside the cell is artificially modified, and the character of the cell is mutated. Wild-type cyanobacteria are also referred to as parent cyanobacteria. The method for modifying the genomic DNA of a wild-type cyanobacterium is not limited to a specific method. Examples of such methods are genetic recombination, genome editing, and mutagenesis. For example, as a method for modifying the genomic DNA of cyanobacteria, a gene recombination method by natural transformation (see Document A below) or a genome editing method using a CRISPR-Cpf1 system (see Document B below) can be used. Document A: Hirose et al., Photosynthesis Research Methods, Cryogenic Science, Vol. 67, Institute for Cryogenic Science, Hokkaido University, 2008, pp. 9-15, ISSN: 1880-7593, http://hdl.handle.net/2115/39084 Document B: Ungerer et al., 2016, Scientific Reports, 6: 39681

In the modified cyanobacterium, for example, the function of a protein involved in the binding between the outer membrane and the cell wall is suppressed or lost. Thus, in the modified cyanobacteria, the binding between the cell wall and the outer membrane is partially reduced. In other words, the binding amount and the binding force in the binding between the cell wall and the outer membrane are reduced. Therefore, the outer membrane is likely to be partially detached from the cell wall. As a result, in the modified cyanobacteria, intracellular products, which are proteins and metabolites produced in the cells, are likely to leak out of the cells. As a result, proteins and metabolites produced in the cells of the modified cyanobacteria are easily secreted to the outside of the cells, and intracellular products can be collected as secretions without performing an extraction treatment of intracellular products such as crushing the cells. As a result, the physiological activity and yield of the intracellular product secreted to the outside of the bacterial cell are less likely to decrease. Therefore, among the intracellular products of the modified cyanobacterium, the physiological activity and yield of a substance that can contribute to having at least one effect selected from the group consisting of an antioxidant effect and an ultraviolet absorbing effect are less likely to decrease. As a result, the antioxidant can more advantageously have at least one effect selected from the group consisting of an antioxidant effect and an ultraviolet absorbing effect.

The gene modified in the modified cyanobacterium is not limited to a particular gene. In the modified cyanobacterium, a gene encoding at least one of a surface layer homology (SLH) domain-retaining outer membrane protein or a cell wall-pyruvate modifying enzyme, which are involved in binding between an outer membrane and a cell wall, is modified. As a result, the binding between the SLH domain of the SLH domain-retaining outer membrane protein of the outer membrane of the modified cyanobacterium and the covalently bound sugar chain on the surface of the cell wall is likely to be weakened, and as described above, the outer membrane is likely to be partially detached from the cell wall. Weakening binding means decrease in the amount and strength of the binding.

The gene that is modified in the modified cyanobacterium is, for example, slr1841, slr0688, or slr1908 included in Synechocystis sp. PCC 6803. Alternatively, the gene is a gene corresponding to slr1841, slr0688, or slr1908 of cyanobacteria of the genus Synechococcus, Anabaena, or Nostoc. Slr1841 and slr1908 encode an SLH domain-retaining outer membrane protein. The protein consists of a C-terminal region embedded in the lipid membrane constituting the outer membrane of cyanobacteria and an N-terminal SLH domain protruding from the lipid membrane, and is widely distributed in cyanobacteria (see Document C below). The region embedded in the lipid membrane forms a channel for allowing permeation of hydrophilic substances through the outer membrane. On the other hand, the SLH domain has a function of binding to a cell wall (see Document D below). In order for the SLH domain to bind to the cell wall, the covalently linked sugar chain in peptidoglycan needs to be modified with pyruvic acid (see Document E below). A cell wall-pyruvate modification enzyme, which is an enzyme catalyzing a pyruvate modification reaction of a covalently linked sugar chain in peptidoglycan, has been identified in Bacillus anthracis, which is a gram-positive bacterium, and named CsaB (see Document F below). The cell wall-pyruvate modifying enzyme may be encoded by slr0688.
Document C: Kojima et al., 2016, Biosci. Biotech. Biochem., 10 : 1954-1959
Document D: Kowata et al., 2017, J. Bacteriol., 199 : e00371-17
Document E: Kojima et al., 2016, J. Biol. Chem., 291 : 20198-20209
Document F: Mesnage et al., 2000, EMBO J., 19: 4473-4484

The above-mentioned antioxidant can be produced by, for example, a method including causing cyanobacteria to secrete a secretion to obtain a component having an antioxidant effect.

FIG. 1 is a flowchart showing an example of a method for producing an antioxidant. As shown in FIG. 1, in step S1, cyanobacteria capable of secreting secretions contained in an antioxidant are prepared. In the preparation of the cyanobacteria, the conditions of the cyanobacteria are adjusted to allow secretion. For example, step S1 may include restoring bacterial cells from a lyophilized product or a glycerol stock of cyanobacteria, or may include collecting cyanobacteria that have completed secreting their secretions in step S2, which will be described later. The prepared cyanobacteria may be modified cyanobacteria as described above. In this case, step S1 may include, for example, genetically modifying a parent cyanobacterium to produce a modified cyanobacterium, or may include restoring bacterial cells from a lyophilized product or a glycerol stock of the modified cyanobacterium. Step S1 may include collecting the modified cyanobacteria that have completed secreting their secretions in step S2, which will be described later.

The modified cyanobacteria are, for example, genetically modified by a vector DNA. A vector DNA is, for example, a plasmid or virus designed for genetic modification. The vector DNA contains, for example, a gene sequence of a homologous region at a site targeted for genetic modification, a gene sequence for suppressing expression of a target of genetic modification, and a gene sequence functioning as a marker for genetic modification.

FIG. 2 is a diagram schematically showing an example of a gene sequence of a vector DNA used for genetic modification of cyanobacteria. The site targeted for genetic modification using this vector DNA is the slr0688 gene of cyanobacteria. A homologous region 1 (see SEQ ID NO: 1), which is a sequence immediately upstream of the transcription initiation site of the slr0688 gene, and a homologous region 2 (see SEQ ID NO: 2), which is a sequence immediately downstream of the transcription initiation site of the slr0688 gene, are introduced into the vector DNA. In the vector DNA, the sequence for suppressing the expression of the slr0688 gene is the sequence of the promoter portion of the petE gene of cyanobacteria (see SEQ ID NO: 3). This sequence makes it possible to suppress the expression of the slr0688 gene. The vector DNA is provided with the antibiotic kanamycin resistance gene (see SEQ ID NO: 4) as a marker for genetic modification. This gene serves as an indicator for selecting modified cyanobacteria into which a sequence has been newly introduced by homologous recombination. The vector DNA comprises the mazF gene of E. coli (see SEQ ID NO: 5) as a marker for genetic modification. This gene acts as a lethal gene and serves as an indicator for selecting modified cyanobacteria in which an existing sequence is deleted by homologous recombination. This sequence for regulating the gene is a sequence (see SEQ ID NO: 6) of the promoter part of a nrsB gene of the cyanobacterial. The presence or absence of expression of the lethal gene can be arbitrarily regulated by this sequence.

The method for constructing the vector DNA is not particularly limited. This vector DNA can be constructed, for example, by artificial gene synthesis. For example, genomic DNA is extracted from cells of the parent cyanobacteria. From the genomic DNA, a sequence corresponding to a homologous region at a site targeted for genetic modification is amplified by a polymerase chain reaction (PCR) method. This vector DNA can be constructed by introducing the obtained gene fragment into a plasmid DNA. The original plasmid DNA is not limited to a specific DNA. The original plasmid DNA is preferably pUC19. pUC19 is easily introduced into and replicated in E. coli. A method for introducing a gene fragment into a plasmid DNA is not limited to a specific method. The method includes, for example, linearizing plasmid DNAs using an arbitrary restriction enzyme, and then ligating the linearized plasmid DNAs using a DNA Ligation Kit or In-Fusion HD Cloning Kit of Takara Bio Inc. According to this method, a gene fragment can be easily introduced onto a plasmid DNA.

The method for suppressing the expression of a target gene by genetic modification is not limited to a particular method. The method includes, for example, introducing a promoter sequence of a gene having a weaker expression than the target gene or a promoter sequence capable of arbitrarily regulating the presence or absence of expression of the target gene immediately upstream of the target gene.

A promoter is a regulatory sequence involved in the efficiency of the transcription initiation reaction of a gene. A promoter can broadly include both regulatory sequences that act relatively close to the transcription start site and regulatory sequences that act relatively distant from the transcription start site. Examples of promoters capable of regulating the presence or absence of expression in cyanobacteria are the promoter of the petE gene, the promoter of the nirA gene, and the promoter of the psbA2 gene of cyanobacteria (see Document G below). For example, the promoter of the petE gene is a promoter that is expressed only when cells having the promoter are in a growth environment in which Cu²⁺ ions are present (see Document H below).
Document G: Heidorn et al., 2011, Methods in Enzymology, 497, 539-579
Document H: Briggs et al., 1990, Plant Molecular Biology, 15, 633-642

The marker for genetic modification used in genetic modification of cyanobacteria is not limited to a particular marker. As a marker for genetic modification, an antibiotic resistance gene or a lethal gene is desirably used, as described above. By introducing a gene sequence containing an antibiotic resistance gene into a parent cyanobacterium, only the modified cyanobacterium into which the gene sequence is introduced can grow even in the presence of an antibiotic. Therefore, the modified cyanobacteria can be easily selected. The antibiotic resistance gene is not limited to a particular resistance gene. Examples of antibiotic resistance genes are kanamycin, spectinomycin and chloramphenicol resistance genes. By deleting the gene sequence containing the lethal gene from the modified cyanobacterium, only the modified cyanobacterium in which the gene sequence is deleted can grow even under the lethal gene-inducing conditions. Therefore, the modified cyanobacteria can be easily selected. The lethal gene is not limited to a particular gene. Examples of lethal genes are the mazF gene of E. coli and the sacB gene of Bacillus subtilis (see Document I below).
Document I: Cheah et al., 2012, Biotechnol. Prog., 29(1), 23-30

As described above, preferably, a promoter sequence capable of regulating the presence or absence of expression in cyanobacteria is added upstream of the lethal gene. By regulating the expression of the lethal gene with this sequence, the lethal gene is not expressed before the genetic modification, and it is easy to prevent the growth of cyanobacteria from being hindered.

As shown in FIG. 1, in step S2, the cyanobacteria prepared in step S1 are caused to secrete secretions. When the cyanobacterium is the modified cyanobacterium, proteins and metabolites produced in the cell are easily secreted outside the outer membrane. These intracellular products may include substances that contribute to the antioxidant effect of antioxidants. For example, in step S2, by culturing the modified cyanobacterium under predetermined conditions, a substance that contributes to the antioxidant effect of the antioxidant is secreted outside the bacterial cell.

The culture of cyanobacteria can generally be carried out on the basis of a liquid culture using BG-11 medium or a variant thereof. Therefore, cultivation of the modified cyanobacteria may be performed in the same manner. The culture period of cyanobacteria for producing an antioxidant is a period in which proteins and metabolites can be accumulated at high concentrations under conditions in which bacterial cells are sufficiently grown, and may be, for example, 1 to 3 days or 4 to 7 days. Examples of the culture method include aeration stirring culture and shaking culture. When the modified cyanobacteria are cultured under such conditions, the modified cyanobacteria produce intracellular products, which are proteins and metabolites, in the cells, secrete the intracellular products, and excrete the intracellular products into the culture solution. The intracellular products include substances that contribute to the antioxidant effect of the antioxidant. When the secretions released into the culture solution are collected, the culture solution is subjected to filtration or centrifugation to remove solid contents such as cells, namely, bacterial cells, from the culture solution, and the culture supernatant is collected. By such a production method, a substance contributing to the antioxidant effect of the antioxidant is secreted extracellularly from the cyanobacterium. Therefore, it is not necessary to disrupt the cells in order to obtain a substance that contributes to the antioxidant effect of the antioxidant. Therefore, the cyanobacteria remaining after the collection of the secretions can be repeatedly used to produce antioxidants.

The method for collecting the secretions secreted in the culture solution of cyanobacteria is not limited to the above method. For example, the secretion in the culture solution may be collected while cyanobacteria are cultivated. For example, by using a permeable membrane that allows passage of the secretion, the secretion that has passed through the permeable membrane may be collected. In this case, since the secretion in the culture solution can be collected while the cyanobacteria are cultivated, a process of removing the cyanobacterial cells from the culture solution can be made unnecessary. Therefore, the antioxidant can be produced more simply and efficiently.

The antioxidant may be a culture solution itself of cyanobacteria, a culture solution from which cyanobacteria have been removed, or a substance separated from a culture solution of cyanobacteria by a membrane separation method. The antioxidant may be in liquid, cream or powder form. The powdery antioxidant can be produced, for example, by drying a liquid containing secretions by spray-drying.

As described above, since it is not necessary to perform the process of collecting the bacterial cells from the culture solution of the cyanobacteria and the process of disrupting the bacterial cells, it is possible to reduce the damage and stress received by the cyanobacteria. Therefore, the productivity of secretion of the cyanobacteria is less likely to decrease, and the cyanobacteria can be used for a long period of time. Thus, for example, by using the modified cyanobacteria, the antioxidant can be obtained easily and efficiently.

The secretion included in the antioxidant is not limited to a specific compound. The secretion contains, for example, at least one selected from the group consisting of nucleic acids, adenosine, polysaccharides, amino acids, phycocyanin, and mycosporine-like amino acids. This allows the antioxidant to have the desired effect. It is considered that when the secretion contains phycocyanin, the antioxidant capacity of the antioxidant is likely to be high. It is considered that when the secretion contains a mycosporine-like amino acid, the ultraviolet absorbing ability of the antioxidant is likely to increase. It is considered that when the secretion contains phycocyanin, polysaccharide, or amino acid, the antioxidant is likely to have high moisturizing properties. It is considered that when the secretion contains a nucleic acid or adenosine, the antioxidant is likely to have a whitening effect.

The antioxidant may contain components other than the secretions. The antioxidant may comprise, for example, an aqueous component, an oily component, a surfactant, or a fragrance. Examples of aqueous components are water, glycerin, ethanol, BG, PEG, and sodium hyaluronates. Examples of oily components are squalane, mineral oil, petrolatum, and vegetable oils. Examples of the surfactant include a cationic surfactant, an anionic surfactant, a nonionic surfactant, and a zwitterionic surfactant. The antioxidant may contain other components as necessary. The antioxidant may include, for example, sodium nitrate, magnesium sulfate, potassium chloride, or an organic acid having a carboxy group. The antioxidant may be contained in a pharmaceutical product, a quasi-drug, a cosmetic product, a food product, or a beverage product.

The antioxidant can provide a method for removing active oxygen. In this method, active oxygen is removed by the antioxidant. In this case, the antioxidant may be applied to the skin by a method such as application or spraying, or may be taken into the body orally or by injection. Antioxidants may be used on plants.

### (Topical dermatological composition)

The topical dermatological composition according to the present embodiment contains a secretion of cyanobacteria. The topical dermatological composition has at least one effect selected from the group consisting of an antioxidant effect and an ultraviolet absorbing effect. Having an antioxidant effect means that, for example, the degree of the antioxidant effect is larger than that in a case where the topical dermatological composition is not used. Having an ultraviolet absorption effect means that, for example, the degree of the ultraviolet absorption effect is larger than that in a case where the topical dermatological composition is not used.

The topical dermatological composition contains, for example, a secretion excreted to the outside of cyanobacteria, but does not contain cyanobacteria themselves or a component derived from cyanobacteria themselves. In other words, the antioxidant topical dermatological composition contains, for example, as a component derived from cyanobacteria, only secretions excreted to the outside of cyanobacteria.

The topical dermatological composition may further have a moisturizing effect. Having a moisturizing effect means, for example, that the moisturizing effect is exhibited to a greater extent than in a case where the topical dermatological composition is not used. The basic action of moisturizing can be classified into three actions: a "moisturizing action" of supplementing moisture in the stratum corneum; a "softening action" of imparting flexibility to the skin by giving and holding a moisturizing component; and an "evaporation suppressing action" of confining moisture or a moisturizing component in the stratum corneum. The degree of the moisturizing effect can be evaluated, for example, by applying the topical dermatological composition to the skin and then measuring a temporal change in skin moisture content.

The secretions contained in the topical dermatological composition are, for example, secretions of modified cyanobacteria. The modified cyanobacteria are, for example, genetically modified so as to allow this secretion to be excreted outside the cyanobacteria. For example, the modified cyanobacteria can excrete, as secretions, compounds that are difficult to excrete by wild-type cyanobacteria that have not been modified genetically. The modified cyanobacteria are genetically modified in such a way that, for example, a component having at least one effect selected from the group consisting of an antioxidant effect and an ultraviolet absorbing effect is allowed to be excreted.

The above description regarding the cyanobacteria, the secretion of cyanobacteria, the modified cyanobacteria, and the culture of the modified cyanobacteria in the antioxidant also applies to the topical dermatological composition, unless there is a technical contradiction.

The topical dermatological composition can be produced, for example, by a method including causing cyanobacteria to secrete secretions to obtain a component having at least one effect selected from the group consisting of an antioxidant effect and an ultraviolet absorbing effect. The above description of the method for producing the antioxidant also applies to the method for producing the topical dermatological composition unless there is a technical contradiction.

For example, when the modified cyanobacteria are cultured, the modified cyanobacteria produce intracellular products, which are proteins and metabolites, in the cells, secrete the intracellular products, and excrete the intracellular products into the culture solution. The intracellular product contains a substance that contributes to the topical dermatological composition having at least one effect selected from the group consisting of an antioxidant effect and an ultraviolet absorbing effect. When the secretions released into the culture solution are collected, the culture solution is subjected to filtration or centrifugation to remove solid contents such as cells, namely, bacterial cells, from the culture solution, and the culture supernatant is collected. In such a production method, a substance that contributes to the topical dermatological composition having at least one effect selected from the group consisting of an antioxidant effect and an ultraviolet absorbing effect is secreted extracellularly from cyanobacteria. Therefore, it is not necessary to disrupt cells in order to obtain a substance that contributes to the topical dermatological composition having at least one effect selected from the group consisting of an antioxidant effect and an ultraviolet absorbing effect. Therefore, the cyanobacteria remaining after the collection of the secretion can be repeatedly used to produce a topical dermatological composition.

The topical dermatological composition may be a culture solution itself of cyanobacteria, a culture solution from which cyanobacteria have been removed, or a composition separated from a culture solution of cyanobacteria by a membrane separation method. The topical dermatological composition may be in the form of liquid, cream, or powder. The powdery topical dermatological composition can be produced, for example, by drying a liquid containing secretions by spray-drying.

As described above, since it is not necessary to perform the process of collecting the bacterial cells from the culture solution of the cyanobacteria and the process of disrupting the bacterial cells, it is possible to reduce the damage and stress received by the cyanobacteria. Therefore, the productivity of secretion of the cyanobacteria is less likely to decrease, and the cyanobacteria can be used for a long period of time. Thus, for example, by using the modified cyanobacteria, a topical dermatological composition can be obtained easily and efficiently.

The secretion contained in the topical dermatological composition is not limited to a specific compound. The secretion contains, for example, at least one selected from the group consisting of nucleic acids, adenosine, polysaccharides, amino acids, phycocyanin, and mycosporine-like amino acids. This allows the topical dermatological composition to advantageously have at least one effect selected from the group consisting of an antioxidant effect and an ultraviolet absorbing effect. It is considered that when the secretion contains phycocyanin, the antioxidant capacity of the topical dermatological composition is likely to increase. It is considered that when the secretion contains a mycosporine-like amino acid, the ultraviolet absorption ability of the topical dermatological composition is likely to be increased. It is considered that when the secretion contains phycocyanin, polysaccharide, or amino acid, the topical dermatological composition is likely to have high moisturizing properties. It is considered that when the secretion contains a nucleic acid or adenosine, the topical dermatological composition is likely to have a whitening effect.

The topical dermatological composition may contain components other than the secretion. The topical dermatological composition may contain, for example, an aqueous component, an oily component, a surfactant, or a perfume. Examples of aqueous components are water, glycerin, ethanol, BG, PEG, and Na hyaluronates. Examples of oily components are squalane, mineral oil, petrolatum, and vegetable oils. Examples of the surfactant include a cationic surfactant, an anionic surfactant, a nonionic surfactant, and a zwitterionic surfactant. The topical dermatological composition may contain other components as necessary. The topical dermatological composition may contain, for example, sodium nitrate, magnesium sulfate, potassium chloride, or an organic acid having a carboxy group.

In the above-mentioned topical dermatological composition, it is possible to provide a skin care method including applying the topical dermatological composition to the skin. The method for applying the topical dermatological composition to the skin may be application or spraying.

### (Moisturizer)

The moisturizer according to the present embodiment contains a secretion of cyanobacteria. Having a moisturizing effect means, for example, that the degree of the moisturizing effect is larger than that in a case where the moisturizer is not used. The basic action of moisturizing can be classified into three actions: a "moisturizing action" of supplementing moisture in the stratum corneum; a "softening action" of imparting flexibility to the skin by giving and holding a moisturizing component; and an "evaporation suppressing action" of confining moisture or a moisturizing component in the stratum corneum. The degree of the moisturizing effect can be evaluated, for example, by applying the moisturizer to the skin and then measuring the change in skin moisture content over time.

The moisturizer contains, for example, a secretion excreted to the outside of cyanobacteria, but does not contain cyanobacteria itself or a component derived from cyanobacteria itself. In other words, the moisturizer contains, for example, only secretions excreted to the outside of cyanobacteria as components derived from cyanobacteria.

The moisturizer may further have at least one effect selected from the group consisting of an antioxidant effect and an ultraviolet absorbing effect. In the present specification, having an antioxidant effect means that, for example, the degree of the antioxidant effect is larger than that in a case where a moisturizer is not used. In the present specification, having an ultraviolet absorbing effect means that, for example, the degree of the ultraviolet absorbing effect is larger than that in a case where a moisturizer is not used.

The secretions contained in the moisturizer are, for example, secretions of modified cyanobacteria. The modified cyanobacteria are, for example, genetically modified so as to allow this secretion to be excreted outside the cyanobacteria. For example, the modified cyanobacteria can excrete, as secretions, compounds that are difficult to excrete by wild-type cyanobacteria that have not modified genetically. The modified cyanobacteria are, for example, genetically modified to allow a component having a moisturizing effect to be excreted.

The above explanations regarding cyanobacteria, cyanobacterial secretions, modified cyanobacteria and modified cyanobacterial cultures in antioxidants also apply to moisturizers, unless technically contradictory.

The moisturizer can be produced by, for example, a method including causing cyanobacteria to secrete secretions to obtain a component having a moisturizing effect. The above explanations regarding the process for producing the antioxidant also apply to the process for producing the moisturizer, unless there is a technical contradiction.

For example, when the modified cyanobacteria are cultured, the modified cyanobacteria produce intracellular products, which are proteins and metabolites, in the cells, secrete the intracellular products, and excrete the intracellular products into the culture solution. The intracellular product contains a substance that contributes to the moisturizing effect of the moisturizer. When the secretions released into the culture solution are collected, the culture solution is subjected to filtration or centrifugation to remove solid contents such as cells, namely, bacterial cells, from the culture solution, and the culture supernatant is collected. In such a production method, a substance contributing to the moisturizing effect of the moisturizer is secreted outside the cells of the cyanobacteria. Therefore, it is not necessary to crush the cells in order to obtain a substance that contributes to the moisturizing effect of the moisturizer. Therefore, the cyanobacteria remaining after the collection of the secretions can be repeatedly used to produce a moisturizer.

The method for collecting the secretions secreted in the culture solution of cyanobacteria is not limited to the above method. For example, the secretion in the culture solution may be collected while cyanobacteria are cultivated. For example, by using a permeable membrane that allows passage of the secretion, the secretion that has passed through the permeable membrane may be collected. In this case, since the secretion in the culture solution can be collected while the cyanobacteria are cultivated, a process of removing the cyanobacterial cells from the culture solution can be made unnecessary. Therefore, the moisturizer can be produced more simply and efficiently.

The moisturizer may be a culture solution itself of cyanobacteria, may be a culture solution from which cyanobacteria have been removed, or may be a substance separated from a culture solution of cyanobacteria by a membrane separation method. The moisturizer may be in liquid, cream or powder form. The powdery moisturizer can be produced by, for example, drying a liquid containing secretions by spray-drying.

As described above, since it is not necessary to perform the process of collecting the bacterial cells from the culture solution of the cyanobacteria and the process of disrupting the bacterial cells, it is possible to reduce the damage and stress received by the cyanobacteria. Therefore, the productivity of secretion of the cyanobacteria is less likely to decrease, and the cyanobacteria can be used for a long period of time. As described above, for example, by using the modified cyanobacteria, the moisturizer can be easily and efficiently obtained.

The secretion contained in the moisturizer is not limited to a specific compound. The secretion contains, for example, at least one selected from the group consisting of nucleic acids, adenosine, polysaccharides, amino acids, phycocyanin, and mycosporine-like amino acids. This allows the moisturizer to have the desired effect. It is considered that when the secretion contains phycocyanin, the antioxidant capacity of the moisturizer is likely to be increased. It is considered that when the secretion contains a mycosporine-like amino acid, the ultraviolet absorbing ability of the moisturizer is likely to be increased. It is considered that when the secretion contains phycocyanin, polysaccharide, or amino acid, the moisturizer is likely to have high moisturizing properties. It is considered that the moisturizer is likely to have a whitening effect when the secretion contains a nucleic acid or adenosine.

The moisturizer may contain components other than the secretions. The moisturizer may include, for example, an aqueous component, an oily component, a surfactant, or a fragrance. Examples of aqueous components are water, glycerin, ethanol, BG, PEG, and Na hyaluronates. Examples of oily components are squalane, mineral oil, petrolatum, and vegetable oils. Examples of the surfactant include a cationic surfactant, an anionic surfactant, a nonionic surfactant, and a zwitterionic surfactant. The moisturizer may contain other components as necessary. The moisturizer may be a moisturizer that moisturizes the stratum corneum by directly applying moisture to the stratum corneum, or may be an emollient that suppresses evaporation of moisture by a film formed on the surface of the stratum corneum by the moisturizer. The moisturizer may include, for example, sodium nitrate, magnesium sulfate, potassium chloride, or an organic acid having a carboxy group.

With the moisturizer, it is possible to provide a skin care method including applying the moisturizer to the skin. The method for applying the moisturizer to the skin may be application or spraying.

### (SUPPLEMENTARY NOTES)

Based on the above description, the following techniques are disclosed.

### (Technique 1)

An antioxidant comprising a secretion of cyanobacteria.

### (Technique 2)

The antioxidant according to Technique 1, wherein
the antioxidant has an ultraviolet absorbing effect.

### (Technique 3)

The antioxidant according to Technique 1 or 2, wherein
the secretion is a secretion of a modified cyanobacterium genetically modified to allow the secretion to be excreted outside the cyanobacterium.

### (Technique 4)

The antioxidant according to Technique 3, wherein
a function of a protein involved in binding between an outer membrane and a cell wall is suppressed or lost in the modified cyanobacterium.

### (Technique 5)

The antioxidant according to Technique 4, wherein
a gene encoding at least one of a surface layer homology (SLH) domain-retaining outer membrane protein or a cell wall-pyruvate modifying enzyme, both of which are involved in binding between an outer membrane and a cell wall, is modified in the modified cyanobacterium.

### (Technique 6)

The antioxidant according to Technique 5, wherein
the gene is a first gene which is slr1841, slr0688, or slr1908 which is contained in Synechocystis sp. PCC 6803, or a second gene which is a gene corresponding to the first gene, the second gene being contained in a cyanobacterium of a genus Synechococcus, a genus Anabaena, or a genus Nostoc.

### (Technique 7)

The antioxidant according to Technique 3, wherein
the modified cyanobacterium is genetically modified with a vector DNA,
the vector DNA is a plasmid or a virus designed for genetic modification, and
the vector DNA includes a gene sequence of a homologous region at a site targeted by the genetic modification, a gene sequence for suppressing expression of a target of the genetic modification, and a gene sequence functioning as a marker for genetic modification.

### (Technique 8)

The antioxidant according to any one of Technologies 1 to 7, wherein
the secretion comprises at least one selected from the group consisting of a nucleic acid, adenosine, a polysaccharide, an amino acid, phycocyanin, and a mycosporine-like amino acid.

### (Technique 9)

A method for removing active oxygen, comprising:
removing the active oxygen with an antioxidant comprising a secretion of cyanobacteria.

### (Technique 10)

A method for producing an antioxidant agent, comprising:
causing a cyanobacterium to secrete a secretion to obtain an ingredient having a moisturizing effect.

### (Technique 11)

The production method according to Technique 10, wherein
the cyanobacterium is genetically modified to allow the component to be secreted.

### (Technique 12)

A topical dermatological composition, comprising:
a secretion of cyanobacteria, and
having at least one effect selected from the group consisting of an antioxidant effect and an ultraviolet absorbing effect.

### (Technique 13)

The topical dermatological composition according to Technique 12, wherein
the topical dermatological composition further has a moisturizing effect.

### (Technique 14)

The topical dermatological composition according to Technique 12 or 13, wherein
the secretion is a secretion of a modified cyanobacterium genetically modified to allow the secretion to be excreted outside of the cyanobacterium.

### (Technique 15)

The topical dermatological composition according to Technique 14, wherein
a function of a protein involved in binding between the outer membrane and the cell wall is suppressed or lost in the modified cyanobacterium.

### (Technique 16)

The topical dermatological composition according to Technique 15, wherein
a gene encoding at least one of a surface layer homology (SLH) domain-retaining outer membrane protein or a cell wall-pyruvate modifying enzyme, both of which are involved in binding between the outer membrane and the cell wall, is modified in the modified cyanobacterium.

### (Technique 17)

The topical dermatological composition according to Technique 16, wherein
the gene is a first gene which is slr1841, slr0688, or slr1908 which is contained in Synechocystis sp. PCC 6803, or a second gene which is a gene corresponding to the first gene, the second gene being contained in a cyanobacterium of a genus Synechococcus, a genus Anabaena, or a genus Nostoc.

### (Technique 18)

The topical dermatological composition according to Technique 14, wherein
the modified cyanobacterium is genetically modified with a vector DNA,
the vector DNA is a plasmid or a virus designed for genetic modification, and
the vector DNA includes a gene sequence of a homologous region at a site targeted by the genetic modification, a gene sequence for suppressing expression of a target of the genetic modification, and a gene sequence functioning as a marker for genetic modification.

### (Technique 19)

The topical dermatological composition according to any one of Technologies 12 to 18, wherein
the secretion comprises at least one selected from the group consisting of a nucleic acid, adenosine, a polysaccharide, an amino acid, phycocyanin, and a mycosporine-like amino acid.

### (Technique 20)

A skin care method comprising:
applying to skin a topical dermatological composition comprising a secretion of cyanobacteria and having at least one effect selected from the group consisting of an antioxidant effect and an ultraviolet absorption effect.

### (Technique 21)

A method for producing a topical dermatological composition, comprising:
allowing cyanobacteria to secrete a secretion to obtain a component having at least one effect selected from the group consisting of an antioxidant effect and an ultraviolet absorbing effect.

### (Technique 22)

The method for producing the topical dermatological composition according to Technique 21, wherein
the cyanobacterium is genetically modified to allow the component to be secreted.

### (Technique 23)

A moisturizer comprising a secretion of cyanobacteria.

### (Technique 24)

The moisturizer according to Technique 23, wherein
the moisturizer further has at least one effect selected from the group consisting of an antioxidant effect and an ultraviolet absorption effect.

### (Technique 25)

The moisturizer according to Technique 23 or 24, wherein
the secretion is a secretion of a modified cyanobacterium genetically modified to allow the secretion to be excreted outside of the cyanobacterium.

### (Technique 26)

The moisturizer according to Technique 25, wherein
a function of a protein involved in binding between an outer membrane and a cell wall is suppressed or lost in the modified cyanobacterium.

### (Technique 27)

The moisturizer according to Technique 26, wherein
in the modified cyanobacterium, a gene encoding at least one of a surface layer homology (SLH) domain-retaining outer membrane protein and a cell wall-pyruvate modifying enzyme, both of which are involved in binding between an outer membrane and a cell wall, is modified.

### (Technique 28)

The moisturizer according to Technique 27, wherein
the gene is a first gene which is slr1841, slr0688, or slr1908 which is contained in Synechocystis sp. PCC 6803, or a second gene which is a gene corresponding to the first gene, the second gene being contained in a cyanobacterium of a genus Synechococcus, a genus Anabaena, or a genus Nostoc.

### (Technique 29)

The moisturizer according to Technique 25, wherein
the modified cyanobacterium is genetically modified with a vector DNA,
the vector DNA is a plasmid or a virus designed for genetic modification, and
the vector DNA includes a gene sequence of a homologous region at a site targeted by the genetic modification, a gene sequence for suppressing expression of a target of the genetic modification, and a gene sequence functioning as a marker of the genetic modification.

### (Technique 30)

The moisturizer according to any one of Technologies 23 to 29, wherein
the secretion comprises at least one selected from the group consisting of a nucleic acid, adenosine, a polysaccharide, an amino acid, phycocyanin, and a mycosporine-like amino acid.

### (Technique 31)

A method of skin care comprising:
applying a moisturizer to skin,
wherein
the moisturizer comprises a secretion of cyanobacteria.

### (Technique 32)

A method for producing a moisturizer, comprising:
allowing cyanobacteria to secrete a secretion to obtain a component having a moisturizing effect.

### (Technique 33)

The production method according to Technique 32, wherein
the cyanobacterium is genetically modified in such a way that the component is allowed to be secreted.

### EXAMPLES

Hereinafter, the present disclosure will be described in more detail with reference to the examples. The present disclosure is not limited to the following examples.

### (Preparation of Culture Supernatant Solution of Cyanobacteria)

Each of Escherichia coli strain HST08 and wild-type cyanobacterium Synechocystis sp. PCC 6803 (parent cyanobacteria) was cultured in 5 mL of a LB medium for 16 hours. In the LB medium, the concentration of Triton was 1.0% by mass, the concentration of Yeast Extract was 0.5% by mass, and the concentration of NaCl was 1.0% by mass. In addition, the HST08 and the PCC 6803 were cultured in 10 mL of a BG-11 medium for 120 hours. BG-11 medium was prepared as a one liter aqueous solution containing the components shown in Table 1. The sufficiently grown cells derived from 1 mL of the culture solution were subjected to Wizard Genomic DNA Purification Kit of Promega Corporation to extract genomic DNA derived from the cells.

**[Table 1]**

| Added Solution | Addition Amount | Component | Concentration in Added Solution |
|---|---|---|---|
| Solution I | 2mL | Na₂EDTA·2H₂O | 0.5g/L |
| | | ammonium iron (III) citrate | 3g/L |
| | | citric acid | 3g/L |
| Solution II-a | 25mL | NaNO₃ | 60g/L |
| | | MgSO₄ | 3g/L |
| Solution II-b | 25mL | K₂HPO₄ | 1.56g/L |
| Solution III | 2mL | CaCl₂ | 14.3g/L |
| A6 Solution | 1mL | H₃BO₃ | 2.86g/L |
| | | MnCl₂·4H₂O | 1.81g/L |
| | | ZnSO₄·7H₂O | 0.22g/L |
| | | CuSO₄·5H₂O | 0.08g/L |
| | | Na₂MoO₄·H₂O | 0.021g/L |
| | | Co(NO₃)₂·6H₂O | 0.0494g/L |
| | | H₂SO₄ | 1 droplet |
| 1M TES-KOH (pH 7.5) | 10mL | - | |

Next, in order to construct plasmid DNA to be used for gene modification, a total of seven types of DNA fragments (fragments 1 to 7) were amplified by the PCR method. For the PCR method, KOD One PCR Master Mix-Blue-manufactured by Toyobo Co., Ltd. was used. For the fragment 1, a pUC19 plasmid was used as a template, and a replication origin of Escherichia coli and an antibiotic ampicillin resistance gene were amplified using the primer 1 (see SEQ ID NO: 7) and the primer 2 (see SEQ ID NO: 8). Next, for the fragments 2 to 5, the promoter sequence of the petE gene was amplified using the cyanobacteria-derived genomic DNA as a template, and for the fragment 2, using the primer 3 (see SEQ ID NO: 9) and the primer 4 (SEQ ID NO: 10). For the fragment 3, the promoter sequence of the nrsB gene was amplified using the primer 5 (see SEQ ID NO: 11) and the primer 6 (see SEQ ID NO: 12). For the fragment 4, a homologous region 1 of the slr0688 gene was amplified using the primer 7 (see SEQ ID NO: 13) and the primer 8 (see SEQ ID NO: 14). For the fragment 5, a homologous region 2 of the slr0688 gene was amplified using the primer 9 (see SEQ ID NO: 15) and the primer 10 (see SEQ ID NO: 16). For the fragment 6, the mazF gene was amplified using the above-mentioned E. coli-derived genomic DNA as a template and the primer 11 (see SEQ ID NO: 17) and the primer 12 (see SEQ ID NO: 18). For the fragment 7, the antibiotic kanamycin resistance gene was amplified using the pSL2680 plasmid as a template and the primer 13 (see SEQ ID NO: 19) and the primer 14 (see SEQ ID NO: 20).

The seven fragments were joined together using In-Fusion Snap Assembly Master Mix from Clontech. The plasmid DNA constructed in this manner was introduced into E. coli HST08 Premium Competent Cells (Takara Bio Inc.) by a heat shock method. The obtained E. coli was cultured in the presence of 50 µg/mL of antibiotic ampicillin, and cells into which the plasmid DNA had been introduced were selected. The plasmid DNA replicated in this cells was extracted using Wizard Plus SV Minipreps DNA Purification System (Promega) to obtain pNRSmazF-petE0688 plasmid (see FIG. 2) used for genetic modification.

Parent cyanobacterial cells were cultured in 10 mL of BG-11 medium inside a 125 mL Erlenmeyer flask. This culture was performed by irradiating the flask with white LED light of 100 µmol photons m ^{- 2}s ^{- 1} at 30°C. for 120 hours in an artificial climate chamber and shaking the flask at 160rotations per minute (rpm). The culture solution (0.5 mL) of the parent cyanobacterium thus sufficiently grown was mixed with 3 µg of the pNRSmazF-petE0688 plasmid, and homologous recombination for introducing the promoter sequence of the petE gene immediately upstream of the slr0688 gene was performed by a natural transformation method. The transformed cells were grown on BG-11 agar medium for 2 days, and then transferred to an agar medium containing 20 µg/mL of antibiotic kanamycin and grown for 7 days. When the cells were transferred to another agar medium, Immobilon-NC Triton free MCE 0.45 µm 82 mm disc membrane filter (Merck Millipore) was used.

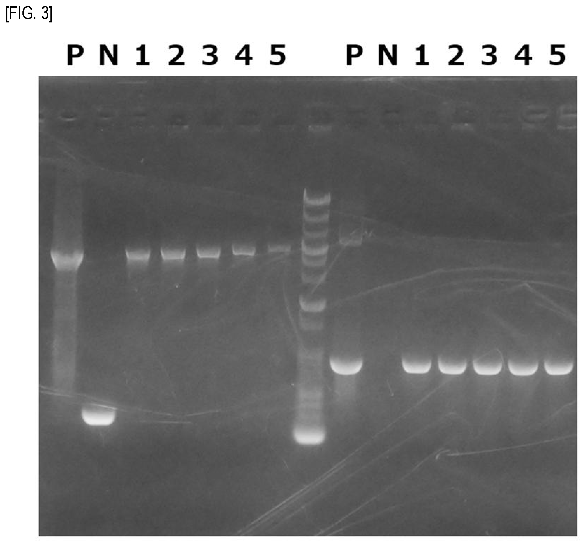

The colonies of the thus-obtained modified cyanobacteria were further transferred to a new agar medium twice and grown to allow homologous recombination to spread throughout the entire genome in the cells. In order to confirm this, colony PCR was performed using the obtained colony as a template. For the colony PCR, GoTaq Green Master Mix (Promega) was used. FIG. 3 shows the results of electrophoresis of the DNA fragments amplified by the colony PCR. The left side of FIG. 3 shows the results of the colony PCR using the primer 15 (see SEQ ID NO: 21) and the primer 16 (see SEQ ID NO: 22). In this colony PCR, amplification was performed from a sequence on the genome located upstream of the site where homologous recombination was performed and a sequence on the genome located downstream of the site. The right side of FIG. 3 shows the results of the colony PCR using the primer 16 and the primer 17 (see SEQ ID NO: 23). In this colony PCR, amplification was performed from the sequence on the genome located downstream of the site where homologous recombination was performed and the sequence in the kanamycin resistance gene where homologous recombination was performed. The middle lane of FIG. 3 shows the result of electrophoresis using Gene Ladder Wide 2 (Nippon Gene Co., Ltd.) as a DNA size marker for the electrophoresis. For the results of the electrophoresis of the DNA fragments in each of the left and right lanes, the numbers 1 to 5 indicate the numbers of colonies used as templates. In addition, "P" shows the results of electrophoresis of DNA fragments amplified using the pNRSmazF-petE0688 plasmid as a control and "N" shows the results of electrophoresis of DNA fragments amplified using the genomic DNA derived from the parent cyanobacterium as a control. In the results shown in FIG. 3, it was confirmed that homologous recombination had spread over the entire genome, and it is understood that modified cyanobacteria into which the promoter sequence of the petE gene had been introduced were obtained.

The cells of the modified cyanobacteria were cultured in one liter BG-11 medium for 120 hours in the same manner as the parent cyanobacteria. As a control, cells of the parent cyanobacteria were also cultured simultaneously. The obtained culture solution was passed through a Millex -GS Syringe Filter Unit, 0.22 µm, manufactured by Merck Millipore to remove the cells. As a result, a culture supernatant solution according to the inventive example containing secretions of the modified cyanobacteria and a culture supernatant solution according to the comparative example as a culture supernatant solution of cells of the parent cyanobacteria were obtained.

### (Detection of LPS)

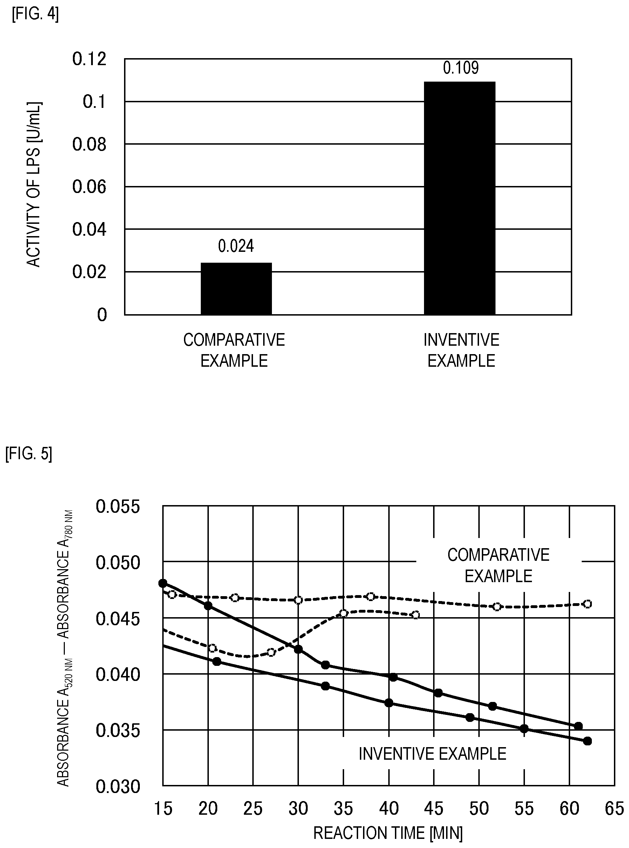

Lipopolysaccharide (LPS), which is a characteristic component indicating detachment of the outer membrane of cyanobacteria, was measured for each of the culture supernatant solution according to the inventive example and the culture supernatant solution according to the comparative example. Specifically, 0.2 mL of each culture supernatant solution was measured for the activity value of LPS using Endospecy ES-24S set and Pyrocolor diazo reagent manufactured by Seikagaku Corporation. FIG. 4 is a graph showing the measurement results of the activity values of LPS in culture supernatants according to the inventive example and the comparative example. As shown in FIG. 4, the LPS activity value of the culture supernatant solution according to the inventive example was higher than the LPS activity value of the culture supernatant solution according to the comparative example, suggesting that the outer membrane was partially detached in the modified cyanobacteria. On the other hand, the activity value of LPS of the culture supernatant solution according to the comparative example was low, and it was suggested that the secretion was not excreted to the outside of the parent cyanobacteria.

### (Measurement of Antioxidant Capacity)

Diphenylpicrylhydrazyl (DPPH) is a stable radical. The antioxidant ability of a test substance can be evaluated by measuring the active oxygen scavenging ability, which is the degree to which the test substance removes unpaired electrons of DPPH. The test substance (150 µL) separated from the culture supernatant liquid according to the inventive example or the culture supernatant liquid according to the comparative example was mixed with 300 µL of the DPPH methanol solution to obtain a sample for evaluation of antioxidant capacity. The culture supernatant solution according to the inventive example and the culture supernatant solution according to the comparative example had a turbidity of 0.4 and 1.9, respectively. The concentration of DPPH in the DPPH methanol solution was 4 mg/L. The elapsed time dependence of the difference obtained by subtracting the absorbance A₇₈₀ₙₘ at a wavelength of 780 nm as a baseline from the absorbance A₅₂₀ₙₘ at a wavelength of 520 nm was measured. The absorbance was measured using an ultraviolet-visible spectrophotometer V-650 manufactured by JASCO Corporation. FIG. 5 is a graph showing the change over time of the difference in absorbance in the samples for evaluation of antioxidant capacity according to the inventive example and the comparative example. In the sample for evaluation of antioxidant capacity using the culture supernatant solution according to the comparative example as a test substance, the change in light absorption due to active oxygen disappeared after about 35 minutes from the start of the reaction, and the antioxidant capacity of the culture supernatant solution according to the comparative example was low. On the other hand, in the sample for evaluation of antioxidative capacity using the culture supernatant solution according to the inventive example as a test substance, light absorption by active oxygen continues to decrease even after 60 minutes have elapsed from the start of the reaction, and the antioxidative capacity of the culture supernatant solution according to the inventive example is very high. It is considered that the culture supernatant solution according to the inventive example contains secretions contributing to high antioxidative capacity.

### (Measurement of Moisturizing properties)

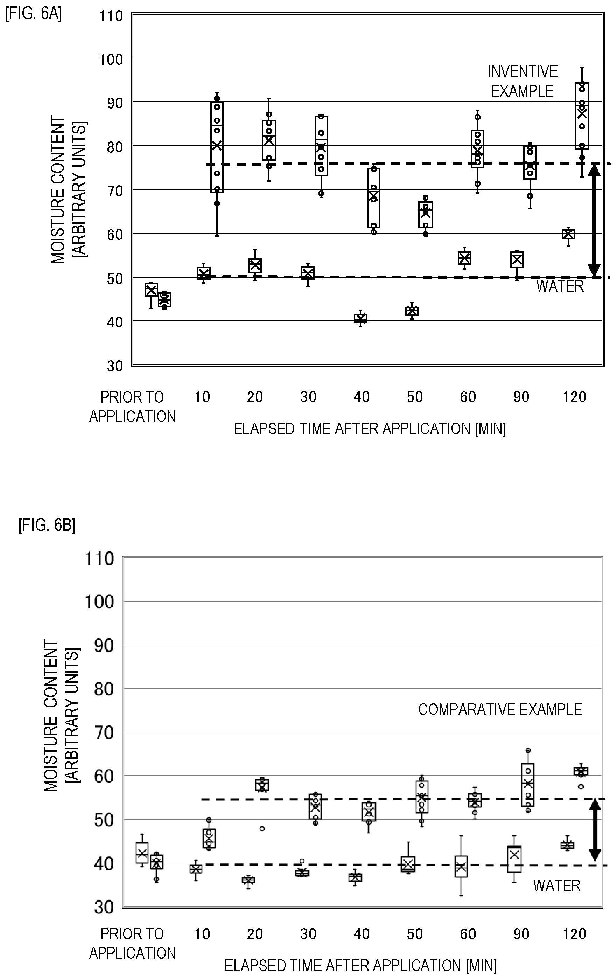

After the oil content on the forearm of the subject was wiped off, the sample for moisturizing properties evaluation according to the inventive example and pure water were dropped on the forearm of the subject in an amount of 20 µL/cm², and the change in skin moisture content over time after natural drying was measured. The sample for moisturizing properties evaluation according to the example was prepared by concentrating the culture supernatant solution according to the example 10-fold by lyophilization. The turbidity of the culture supernatant solution according to Example was 0.4. This measurement was performed 10 times at the same location on the forearm of the subject. Pure water was used as a reference. Corneometer CM825 manufactured by Integral Corporation was used to measure the skin moisture content. In the measurement environment during the test, the relative humidity was 56% and the temperature was 25°C. FIG. 6A is a graph showing a temporal change in skin moisture content when the sample for moisturizing properties evaluation according to the inventive example and pure water are used. The change in skin moisture content over time was measured in the same manner except that the sample for moisturizing properties evaluation according to the comparative example was used instead of the sample for moisturizing properties evaluation according to the inventive example. The sample for moisturizing properties evaluation according to the comparative example was prepared by concentrating the culture supernatant solution according to the comparative example 10 times by lyophilization. The turbidity of the culture supernatant solution according to the comparative example was 1.9. FIG. 6B is a graph showing a temporal change in skin moisture content when the sample for moisturizing properties evaluation according to the comparative example and pure water are used. Broken lines shown in FIGS. 6A and 6B indicate average values of measurement values after 30 minutes have passed since the sample and pure water were dropped on the forearm of the subject. In the comparison between FIG. 6A and FIG. 6B, the sample for moisturizing properties evaluation according to the inventive example exhibited higher moisturizing properties than the sample for moisturizing properties evaluation according to the comparative example. It was suggested that the secretion of the modified cyanobacteria contained in the culture supernatant solution according to the inventive example contains a component that can contribute to having a moisturizing effect.

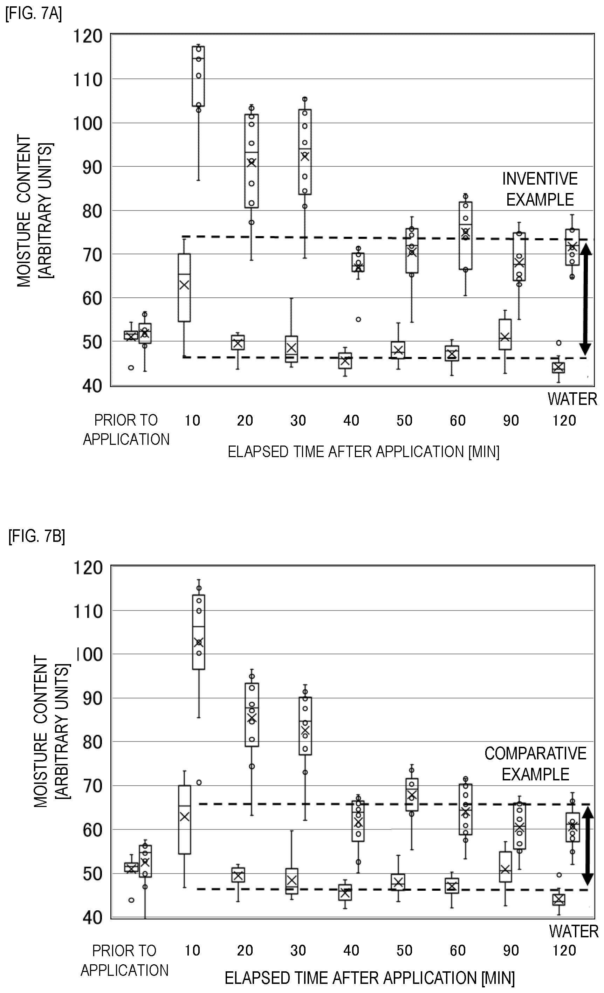

Using the sample for moisturizing properties evaluation according to the inventive example and pure water and the sample for moisturizing properties evaluation according to the comparative example and pure water, the temporal change in skin moisture content was measured on another day in the same manner as the above measurement. In the measurement environment during the test, the relative humidity was 70% and the temperature was 26°C. FIG. 7A is a graph showing a temporal change in skin moisture content when the sample for moisturizing properties evaluation according to the inventive example and pure water are used. FIG. 7B is a graph showing a temporal change in skin moisture content when the sample for moisturizing properties evaluation according to the comparative example and pure water are used. The broken lines shown in FIGS. 7A and 7B indicate the average values of the measurement values 30 minutes after the sample and pure water were dropped on the forearm of the subject. In the comparison between FIG. 7A and FIG. 7B, even in the high humidity environment, the sample for moisturizing properties evaluation according to the inventive example exhibited higher moisturizing properties than the sample for moisturizing properties evaluation according to the comparative example.

### (Evaluation of Ultraviolet Absorptivity)

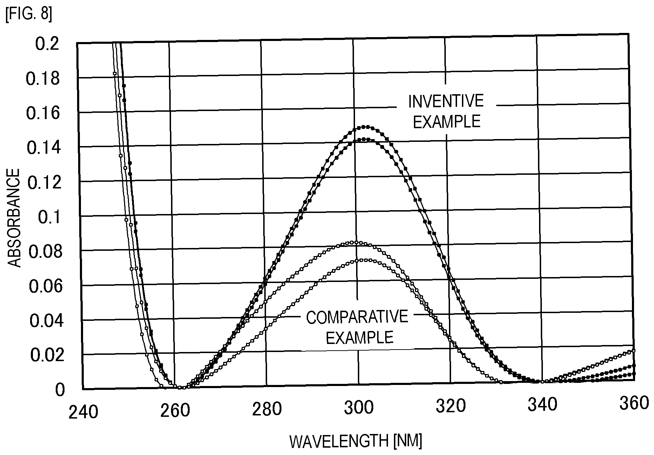

The absorbance of the culture supernatant solution according to the inventive example and the culture supernatant solution according to the comparative example was measured in the vicinity of a wavelength of 300 nm, and the ultraviolet UV-B absorption capacity was evaluated. The turbidity of the culture supernatant solution according to the inventive example was 0.4, and the turbidity of the culture supernatant solution according to the comparative example was 1.9. The absorbance was measured using an ultraviolet-visible spectrophotometer V-650 manufactured by JASCO Corporation. In order to evaluate the UV-B absorption ability, correction was performed in which a straight line connecting a wavelength of 260 nm and a wavelength of 340 nm in the absorption spectrum was treated as a background. FIG. 8 is a graph showing ultraviolet absorption spectra of culture supernatant liquids according to the inventive example and the comparative example. This graph was obtained by the above correction. As shown in FIG. 8, the culture supernatant solution according to the inventive example exhibited about twice the absorbance at a wavelength of 300 nm as compared with the culture supernatant solution according to the comparative example. It was suggested that the secretion of the modified cyanobacteria contained in the culture supernatant solution according to the inventive example contains a component that can contribute to having an ultraviolet absorbing effect.

### (Evaluation of High Temperature Tolerance and Stress Tolerance of Plants)

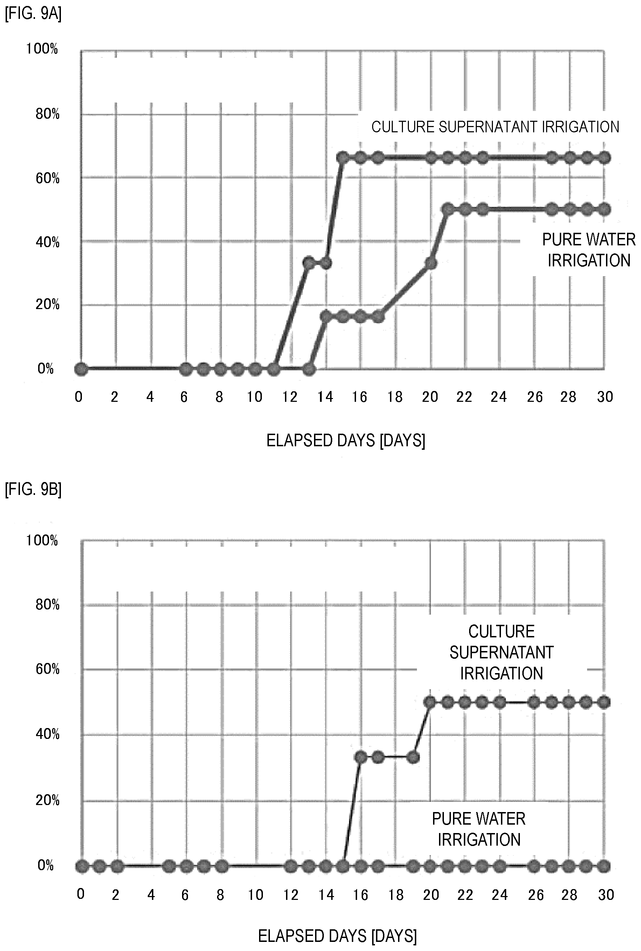

Pansy seeds were sown in commercially available rock wool to prepare 12 pieces of rock wool sown with the pansy seeds. Thereafter, 1 mL of the culture supernatant solution according to the example was irrigated to six rock wools at the time of sowing. In addition, 1 mL of pure water was irrigated to the remaining 6 rock wools at the time of sowing. Each rock wool sown with the pansy seeds was placed in an environment with a temperature of 40°C and an illuminance of 40,000 lux for 6 hours every day. After sowing the pansy seeds, the presence or absence of the development of pansy cotyledons was checked every day. Thus, the cotyledon development ratio of the pansies sown in the rock wool irrigated with the culture supernatant solution according to the inventive example and the cotyledon development ratio of the pansies sown in the rock wool irrigated with pure water were determined. The cotyledon development ratio is a ratio of the number of the rock wools in which the development of the cotyledons was confirmed to the number of the rock wools in which the pansy seeds were sown. The results are shown in FIG. 9A.

Seeds of spinach (variety: Mirage) were sown in commercially available rock wool to prepare 12 rock wools sown with the spinach seeds. Thereafter, 1 mL of the culture supernatant solution according to the inventive example was irrigated to six rock wools at the time of sowing. In addition, 1 mL of pure water was irrigated to the remaining 6 rock wools at the time of sowing. Each rock wool sown with the spinach seeds was placed in an environment with a temperature of 40°C and an illuminance of 40,000 lux for 6 hours every day. After sowing the spinach seeds, the development of spinach cotyledons was checked every day. Thus, the cotyledon development ratio of spinach sown in the rock wool irrigated with the culture supernatant solution according to the inventive example and the cotyledon development ratio of spinach sown in the rock wool irrigated with pure water were determined. The cotyledon development ratio is a ratio of the number of the rock wools in which the development of the cotyledons was confirmed to the number of the rock wools in which the spinach seeds were sown. The results are shown in FIG. 9B.

As shown in FIGS. 9A and 9B, the cotyledon development ratio in the rock wool irrigated with the culture supernatant solution according to the inventive example was higher than the cotyledon development ratio in the rock wool irrigated with pure water for 30 days after sowing. It was suggested that high temperature tolerance and stress tolerance of plants such as pansy and spinach are enhanced by using the culture supernatant solution according to the inventive example.

### INDUSTRIAL APPLICABILITY

The antioxidant of the present disclosure can be used in applications such as pharmaceuticals, quasi-drugs, cosmetics, foods, beverages, and improvement of high temperature tolerance and stress tolerance of plants.

## Claims

1. An antioxidant comprising a secretion of cyanobacteria.

2. The antioxidant according to claim 1, wherein
the antioxidant has an ultraviolet absorbing effect.

3. The antioxidant according to claim 1, wherein
the secretion is a secretion of a modified cyanobacterium genetically modified to allow the secretion to be excreted outside of the cyanobacterium.

4. The antioxidant according to claim 3, wherein
a function of a protein involved in binding between an outer membrane and a cell wall is suppressed or lost in the modified cyanobacterium.

5. The antioxidant according to claim 4, wherein
in the modified cyanobacterium, a gene encoding at least one of a surface layer homology (SLH) domain-retaining outer membrane protein and a cell wall-pyruvate modifying enzyme, both of which are involved in binding between the outer membrane and the cell wall, is modified.

6. The antioxidant according to claim 5, wherein
the gene is a first gene which is slr1841, slr0688, or slr1908 which is contained in Synechocystis sp. PCC 6803, or a second gene which is a gene corresponding to the first gene, the second gene being contained in a cyanobacterium of a genus Synechococcus, a genus Anabaena, or a genus Nostoc.

7. The antioxidant according to claim 3, wherein
the modified cyanobacterium is genetically modified with a vector DNA,
the vector DNA is a plasmid or a virus designed for genetic modification, and
the vector DNA comprises a gene sequence of a homologous region at a site targeted by the genetic modification, a gene sequence for suppressing expression of a target of the genetic modification, and a gene sequence functioning as a marker for genetic modification.

8. The antioxidant of claim 1, wherein
the secretion comprises at least one selected from the group consisting of nucleic acids, adenosine, polysaccharides, amino acids, phycocyanin, and mycosporine-like amino acids.

9. A method for removing active oxygen, the method comprising:
removing active oxygen with an antioxidant comprising a secretion of cyanobacteria.

10. A method for producing an antioxidant agent, the method comprising:
causing a cyanobacterium to secrete a secretion to obtain a component having an antioxidant effect.

11. The method according to claim 10, wherein
the cyanobacterium is genetically modified to allow the component to be secreted.
